# EUROPEAN PATENT APPLICATION

(11) **EP 4 495 232 A1**
(43) Date of publication of application: **22.01.2025**
(21) Application number: 23770922.5
(22) Date of filing: 17.03.2023
(51) Int. Cl.: C12N 9/26, C11D 3/386, C11D 17/06, C11D 17/08, C12N 1/15, C12N 1/19, C12N 1/21, C12N 5/10, C12N 9/28, C12N 15/10, C12N 15/31, C12N 15/56, C12N 15/63

(54) **ALPHA-AMYLASE VARIANT**

(30) Priority: 18.03.2022 JP 2022043483; 28.12.2022 JP 2022211794
(71) Applicant: Kao Corporation, Tokyo 103-8210 (JP)
(72) Inventor: SHAKU, Mao, Wakayama-shi, Wakayama 640-8580 (JP); HIOKI, Takahiro, Wakayama-shi, Wakayama 640-8580 (JP); TERAI, Mika, Wakayama-shi, Wakayama 640-8580 (JP); KAWAHARA, Akihito, Wakayama-shi, Wakayama 640-8580 (JP)
(74) Representative: Vossius & Partner Patentanwälte Rechtsanwälte mbB
(86) International application number: PCT/JP2023/010681
(87) International publication number: WO 2023/176970

(57) **Abstract**

Provided is an α-amylase which has excellent amylolytic activity at a low temperature and further has excellent stability at a low pH and/or in the presence of a chelating agent. A variant of a parent α-amylase, comprising an amino acid residue substitution at a position corresponding to position H238 or E185 of an amino acid sequence set forth in SEQ ID NO: 2, and an amino acid residue substitution shown in the following (A) or (B), the parent α-amylase or α-amylase variant having at least 80% sequence identity to the amino acid sequence set forth in SEQ ID NO: 2 (excluding α-amylase variants having amino acid residue substitutions at three positions corresponding to positions H238, S239, and G178, three positions corresponding to positions H238, R209, and G178 and three positions corresponding to positions E185, N190, and G178 of the same amino acid sequence): (A) an amino acid residue substitution at one or more positions selected from the group consisting of positions corresponding to positions T116, A181, A199, A275, V277, A286 and L323 of the amino acid sequence set forth in SEQ ID NO: 2; or (B) an amino acid residue substitution at a position corresponding to position G178 of the amino acid sequence set forth in SEQ ID NO: 2 and one or more amino acid residue substitutions at one or more positions selected from the group consisting of positions corresponding to positions N126, T129, N190, R209 and S239 of the same amino acid sequence.

## Description

### Field of the Invention

The present invention relates to an α-amylase variant.

### Background of the Invention

α-amylases are used in a wide range of industries such as starch, brewing, textiles, pharmaceuticals and food, are known to have suitability for incorporation into cleaning agents, and are incorporated into dishwashing cleaning agents for automatic dishwashers and clothing cleaning agents as components removing starch stains.

Known α-amylases useful for cleaning agents are Bacillus sp. KSM-1378 (FERM BP-3048) strain-derived α-amylase AP1378 (Patent Literature 1), Termamyl and Duramyl (registered trademarks), which are *Bacillus licheniformis*-derived α-amylases, Bacillus sp. DSM12649 strain-derived α-amylase AA560 (Patent Literature 2), Bacillus sp. SP722 strain-derived α-amylase SP722 (SEQ ID NO: 4 of Patent Literature 3), Cytophaga-derived α-amylase CspAmy2 (Patent Literature 4), and the like. With regard to these α-amylases, variants modified to improve their functions for specific applications, for example, variants with enhanced stability in cleaning agents, have been reported (Patent Literature 5).

In recent years, from the viewpoint of environmental protection and cleaning cost reduction, it is important to lower temperatures in dishwashing and laundry washing, particularly in laundry washing in laundries. In addition, the shortening of the cleaning time is also desired. However, the optimum temperatures of most enzymes, including amylases, are higher than temperatures generally set for low-temperature cleaning. For this reason, it is difficult to completely remove many starch stains. Therefore, it is important to find α-amylases which maintain cleaning performance and amylolytic activity even at a a low temperature, and have a high stain removal effect.

Further, in recent years, it has been known that since cleaning in a low-pH environment does not harm items to be cleaned and is not strong as compared with commonly used alkaline compositions, it is effective to keep items to be cleaned, such as glass and patterned dishes, looking new for longer, reduces odors on fabrics, aids in the release of calcium soaps which tend to trap stains on fabrics, improves performance against pH-sensitive stains, and further benefits the feel of fabrics.

Further, dishwashing cleaning agents are not only used to clean dishes, which are main cleaning targets, but also used to clean stainless steel and plastic sinks, so that they may be incorporated with high concentrations of chelating agents such as citric acid, which have detergency against limescale stains.

Therefore, acidic and/or chelating agent-incorporated cleaning agents are considered to have various advantages in cleaning situations, and it is expected that the detergency can be further improved by adding an enzyme such as an α-amylase to such cleaning agents.

Patent Literature 6 discloses an amylase which is stable in a starch liquefaction process under acidic conditions and an amylase which is stable in a chelating agent-incorporated cleaning agent. Further, Patent Literature 7 discloses SP722 α-amylase variant which is stable in a chelating agent-incorporated cleaning agent. A cleansing composition comprising α-amylase YR288, which maintains cleaning performance and amylolytic activity at a a low temperature, and has a high stain removal effect has been reported (Patent Literature 8).

However, there are still problems in providing α-amylases which have high stability in addition to having high detergency in acidic or chelating agent-containing cleaning agents, particularly acidic and chelating agent-containing cleaning agents.

[Patent Literature 1] WO 94/26881
[Patent Literature 2] WO 00/60060
[Patent Literature 3] WO 06/002643
[Patent Literature 4] WO 2014/164777
[Patent Literature 5] WO 98/044126
[Patent Literature 6] JP-A-2021-505167
[Patent Literature 7] JP-B-6185243
[Patent Literature 8] WO 2022/017728

### Summary of the Invention

The present invention relates to the following:
1) A variant of a parent α-amylase, comprising an amino acid residue substitution at a position corresponding to position H238 or E185 of an amino acid sequence set forth in SEQ ID NO: 2, and an amino acid residue substitution shown in the following (A) or (B),
   the parent α-amylase or α-amylase variant having at least 80% sequence identity to the amino acid sequence set forth in SEQ ID NO: 2 (excluding α-amylase variants having amino acid residue substitutions at three positions corresponding to positions H238, S239, and G178, three positions corresponding to positions H238, R209, and G178, and three positions corresponding to positions E185, N190, and G178 of the same amino acid sequence):
   (A) one or more amino acid residue substitutions at one or more positions selected from the group consisting of positions corresponding to positions T116, A181, A199, A275, V277, A286, and L323 of the amino acid sequence set forth in SEQ ID NO: 2; or
   (B) an amino acid residue substitution at a position corresponding to position G178 of the amino acid sequence set forth in SEQ ID NO: 2, and one or more amino acid residue substitutions at one or more positions selected from the group consisting of positions corresponding to positions N126, T129, N190, R209, and S239 of the same amino acid sequence.
2) A polynucleotide encoding the variant according to 1).
3) A vector or DNA fragment comprising the polynucleotide according to 2).
4) A transformed cell comprising the vector or DNA fragment according to 3).
5) A cleansing composition comprising the variant according to 1).

### Detailed Description of the Invention

The present invention relates to providing an α-amylase which exhibits high amylolytic activity at a low temperature and further having excellent stability at a low pH and/or in the presence of a chelating agent.

The present inventors have succeeded in obtaining an α-amylase variant having enhanced stability at a low pH and/or in the presence of a chelating agent by using an α-amylase which functions at a low temperature as a parent, in comparison with the parent α-amylase.
according to the present invention, it is possible to provide an α-amylase variant having enhanced stability at a low pH and/or in the presence of a chelating agent, in comparison with a parent α-amylase. By using such an α-amylase variant, cleaning stably exhibiting a starch stain removal effect at a low pH and further in the presence of a chelating agent, can be carried out.

In the present specification, "amylase" (EC3.2.1.1; α-D-(1→4)-glucan glucanohydrolase) refers to a group of enzymes which catalyze the hydrolysis of starch and other linear or branched 1,4-glycoside oligosaccharides or polysaccharides. The α-amylase activity can be determined by measuring the amount of reducing ends produced by the enzymatic degradation of starch. Further, the determination method is not limited thereto; for example, the α-amylase activity can also be determined by measuring the release of dye by the enzymatic degradation of dye-crosslinked starch such as Phadebas (Soininen, K., M. Ceska, and H. Adlercreutz. "Comparison between a new chromogenic α-amylase test (Phadebas) and the Wohlgemuth amyloclastic method in urine." Scandinavian journal of clinical and laboratory investigation 30.3 (1972): 291-297.).

In the present specification, the identity of amino acid sequences or nucleotide sequences is calculated by the Lipman-Pearson method (Science, 1985, 227: 1435-1441). Specifically, the identity is calculated by analysis using a homology analysis (Search homology) program of genetic information processing software GENETYX Ver. 12 at a unit size to compare (ktup) of 2.

In the present specification, the "amino acid residues" refer to 20 amino acid residues constituting protein, i.e., alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

In the present specification, amino acid positions and variants are denoted as shown below using the officially recognized IUPAC one-letter amino acid abbreviations.

An amino acid at a predetermined position is denoted as [amino acid, position]. For example, threonine at position 226 is denoted as "T226."

"Substitution" of an amino acid is denoted as [original amino acid, position, substituted amino acid]. For example, substitution of threonine at position 226 with alanine is expressed as "T226A."

"Deletion" of an amino acid is denoted as [original amino acid, position, Δ]. For example, deletion of serine at position 181 is expressed as "S181Δ."

Variants including multiple modifications are denoted by using the addition symbol ("+"). For example, "R170Y+G195E" represents substitution of arginine at position 170 with tyrosine and substitution of glycine at position 195 with glutamic acid, respectively.

When it is possible to introduce different modifications at one position, the different modifications are divided by the diagonal ("/"). For example, "R170Y/E" represents substitution of arginine at position 170 with tyrosine or glutamic acid.

In the present specification, the "operable linkage" between a regulatory region such as a promoter and a gene means that the gene and the regulatory region are linked so that the gene can be expressed under the control of the regulatory region. Procedures for the "operable linkage" between the gene and the regulatory region are well known to a person skilled in the art.

In the present specification, the "upstream" and "downstream" relating to a gene refer to the upstream and downstream in the transcription direction of the gene. For example, "a gene located downstream of a promoter" means that the gene is present on the 3' side of the promoter in the DNA sense strand, and the upstream of a gene means a region on the 5' side of the gene in the DNA sense strand.

In the present specification, the term "original" used for a function, property, or trait of a cell is used to indicate that the function, property, or trait is inherent in the cell. In contrast, the term "foreign" is used to describe a function, property, or trait that is introduced from outside the cell, rather than being inherent in the cell. For example, a "foreign" gene or polynucleotide is a gene or polynucleotide introduced into the cell from outside. The foreign gene or polynucleotide may be derived from an organism of the same species as the cell into which the foreign gene or polynucleotide is introduced, or from an organism of a different species (i.e., heterologous gene or polynucleotide).

### <Variant>

The variant of the present invention is a variant of a parent α-amylase having an amino acid residue substitution at a position corresponding to position H238 or E185 of an amino acid sequence set forth in SEQ ID NO: 2, and an amino acid residue substitution shown in the following (A) or (B), and the parent α-amylase or α-amylase variant has at least 80% sequence identity to the amino acid sequence set forth in SEQ ID NO: 2. However, α-amylase variants having amino acid residue substitutions at three positions corresponding to positions H238, S239, and G178, three positions corresponding to positions H238, R209, and G178, and three positions corresponding to positions E185, N190 and G178 of the same amino acid sequence, are excluded.
(A) one or more amino acid residue substitutions at one or more positions selected from the group consisting of positions corresponding to positions T116, A181, A199, A275, V277, A286, and L323 of the amino acid sequence set forth in SEQ ID NO: 2; or
(B) an amino acid residue substitution at a position corresponding to position G178 of the amino acid sequence set forth in SEQ ID NO: 2, and one or more amino acid residue substitutions at one or more positions selected from the group consisting of positions corresponding to positions N126, T129, N190, R209, and S239 of the same amino acid sequence.

That is, the "variant" means a polypeptide having α-amylase activity in which an amino acid residue substitution at a position corresponding to position H238 or E185 of the amino acid sequence set forth in SEQ ID NO: 2, and an amino acid residue substitution shown in the above (A) or (B) occur in combination in the parent α-amylase. The substitution of amino acid residues at such predetermined positions is a modification for enhancing the stability at a low pH and/or in the presence of a chelating agent. Therefore, such a variant has enhanced stability at a low pH and/or in the presence of a chelating agent in comparison with the parent α-amylase.

The "parent α-amylase" means a standard α-amylase to be modified for producing the variant of the present invention and is an α-amylase having at least 80% sequence identity to the amino acid sequence set forth in SEQ ID NO: 2 in the present invention. The parent may be a natural (wild-type) polypeptide or a variant thereof.

Examples of such a parent α-amylase include those preferably having at least 90%, more preferably at least 95%, more preferably at least 96%, more preferably at least 97%, more preferably at least 98%, and more preferably at least 99% sequence identity to the amino acid sequence set forth in SEQ ID NO: 2.
here, the α-amylase consisting of the amino acid sequence set forth in SEQ ID NO: 2 is (R178Δ+T180Δ) α-amylase having deletion of amino acid residues corresponding to R178 and T180 in the amino acid sequence (SEQ ID NO: 4) constituting α-amylase YR288 (WO 2022/017728) registered as WP_100346362.1 in the NCBI protein sequence database. Such an α-amylase has significantly enhanced stability in cleaning agents, in comparison with YR288 (Japanese Patent Application No. 2021-135746). Therefore, any of α-amylase variants having deletion of two or more amino acid residues selected from the group consisting of positions corresponding to positions R178, G179, T180, and G181 of the amino acid sequence set forth in SEQ ID NO: 4, in the amino acid sequence set forth in SEQ ID NO: 2 or an amino acid sequence having at least 90%, preferably at least 95%, more preferably at least 96%, more preferably at least 97%, more preferably at least 98%, and more preferably at least 99% identity thereto, including an α-amylase consisting of the amino acid sequence set forth in SEQ ID NO: 2, can serve as parent α-amylases for the variant of the present invention.

Examples of deletion of amino acid residues at two or more positions preferably include R178Δ+T180Δ, G179Δ+T180Δ, R178Δ+G179Δ, R178Δ+G181Δ, G179Δ+G181Δ, and the like; and more preferably R178Δ+T180Δ.

The mutation positions in the α-amylase variant of the present invention are numbered based on the amino acid numbers of the amino acid sequence set forth in SEQ ID NO: 2.

Other examples of α-amylases consisting of an amino acid sequence having at least 80% identity to the amino acid sequence set forth in SEQ ID NO: 2 include DE0178, which is *Bacillus flexus*-derived α-amylase, RU2C, which is Bacillus sp.-derived α-amylase, and the like (Japanese Patent Application No. 2020-121626).

In the present invention, the "corresponding position" on the amino acid sequence can be determined by aligning the target sequence and the reference sequence (the amino acid sequence set forth in SEQ ID NO: 2 in the present invention) so as to give the maximum homology. Alignment of the amino acid sequences can be carried out by using known algorithms, and the procedure thereof is known to a person skilled in the art. For example, alignment can be carried out by using the Clustal W multiple alignment program (Thompson, J.D. et al., 1994, Nucleic Acids Res. 22: 4673-4680) with default settings. Alternatively, Clustal W2 and Clustal omega, which are revised versions of Clustal W, can also be used. Clustal W, Clustal W2, and Clustal omega are available on the website of, for example, the European Bioinformatics Institute (EBI [www.ebi.ac.uk/index.html]) or the Japan DNA Data Bank operated by National Institute of Genetics (DDBJ [www.ddbj.nig.ac.jp/searches-j.html]). A position in the target sequence that is aligned to any position in the reference sequence by the above alignment is regarded as the "position corresponding" to any position.

The person skilled in the art can further refine the alignment of the amino acid sequences obtained above to optimize them. Such optimal alignment is preferably determined in consideration of the similarity of amino acid sequences, the frequency of inserted gaps, and the like. The similarity of amino acid sequences as mentioned herein refers to, when two amino acid sequences are aligned, the ratio (%) of the number of positions where the same or similar amino acid residues are present in both sequences to the number of full-length amino acid residues. Similar amino acid residues refer to, among the 20 amino acids constituting protein, amino acid residues which have similar properties to each other in terms of polarity and charge, and which undergo so-called conservative substitution. A group consisting of such similar amino acid residues is well known to the person skilled in the art, and examples include, but are not limited to, arginine and lysine or glutamine; glutamic acid and aspartic acid or glutamine; serine and threonine or alanine; glutamine and asparagine or arginine; leucine and isoleucine; and the like.

"Substitution" of amino acid residues at the above predetermined positions means that an amino acid at a position is substituted with a different amino acid.

In the present invention, the number of substitution sites of amino acid residues can be 2 or more, but is preferably 3 or more, more preferably 2 to 20, and more preferably 3 to 19, from the viewpoint of stability at a low pH and/or in the presence of a chelating agent.

The variant is preferably an α-amylase having at least 80%, preferably at least 85%, more preferably at least 90%, more preferably at least 95%, more preferably at least 96%, more preferably at least 97%, more preferably at least 98%, and more preferably at least 99% identity to the amino acid sequence set forth in SEQ ID NO: 2, from the viewpoint of enhancing cleaning performance and stability at a low pH and/or in the presence of a chelating agent.

The variant may have any number of conservative amino acid substitutions as long as it retains the properties as the above variant.

The amino acid residue substitution shown in (A) is one or more amino acid residue substitutions at one or more positions selected from the group consisting of positions corresponding to positions T116, A181, A199, A275, V277, A286, and L323 of the amino acid sequence set forth in SEQ ID NO: 2, but is preferably substitutions at 2 or more positions, and more preferably 3 to 6 positions, from the viewpoint of stability at a low pH and/or in the presence of a chelating agent.

In addition to the above one or more amino acid residue substitutions at one or more positions selected from the group consisting of positions corresponding to positions T116, A181, A199, A275, V277, A286 and L323, it is preferred to further combine one or more amino acid residue substitutions at 1 or more, preferably 2 or more, more preferably 5 or more, and more preferably 8 or more positions selected from the group consisting of positions corresponding to N126, T129, N190, R209, S239, G178, M197, F203, Y240, E255, Y358, W406 and G474, from the viewpoint of enhancing the cleaning performance and the stability at a low pH and/or in the presence of a chelating agent.

The amino acid residue substitutions shown in (B) are an amino acid residue substitution at a position corresponding to position G178 of the amino acid sequence set forth in SEQ ID NO: 2, and one or more amino acid residue substitutions at one or more positions selected from the group consisting of positions corresponding to positions N126, T129, N190, R209, and S239 of the same amino acid sequence.

Preferred embodiments of the substitution of amino acid residues at positions corresponding to positions H238, E185, T116, A181, A199, A275, V277, A286, L323, G178, N126, T129, N190, R209, S239, M197, F203, Y240, E255, Y358, W406 and G474 in the α-amylase variant of the present invention are as shown below.

That is, H238 is preferably substituted with F (H238F) ;
E185 is preferably substituted with P (E185P);
T116 is preferably substituted with Q (T116Q);
A181 is preferably substituted with T, E or Q (A181T/E/Q) ;
A199 is preferably substituted with E (A199E);
A275 is preferably substituted with N (A275N);
V277 is preferably substituted with T (V277T);
A286 is preferably substituted with V (A286V);
L323 is preferably substituted with F (L323F);
G178 is preferably substituted with H (G178H);,
N126 is preferably substituted with Y (N126Y);
T129 is preferably substituted with I (T129I);
N190 is preferably substituted with F (N190F);
R209 is preferably substituted with L, V or I (R209L/V/I) ;
S239 is preferably substituted with A, Q, D, L, Y, P or H (S239A/Q/D/L/Y/P/H);
M197 is preferably substituted with L, T, A, N, Q, S, V or I (M197L/T/A/N/Q/S/V/I);
F203 is preferably substituted with Y (F203Y);
Y240 is preferably substituted with F (Y240F);
E255 is preferably substituted with T (E255T);
Y358 is preferably substituted with C, M, L or V (Y358C/M/L/V);
W406 is preferably substituted with P (W406P); and
G474 is preferably substituted with A, P, E, S, F, R or K (G474A/P/E/S/F/R/K).

Next, the following Table 1 (Tables 1-1 to 1-3) shows suitable mutation combinations which contribute to the enhancement of stability at a low pH and/or in the presence of a chelating agent. Therefore, variants having at least such mutation combinations are variants which particularly contribute to the enhancement of stability at a low pH and/or in the presence of a chelating agent.

**[Table 1]**

| **Table 1-1** | **Table 1-2** | **Table 1-3** |
|---|---|---|
| **T116Q+H238F** | **T116Q+E185P** | **N126Y+A181Q+H238F** |
| **A181T+H238F** | **A181T+E185P** | **T129I+A181Q+H238F** |
| **A181E+H238F** | **A181E+E185P** | **A181Q+N190F+H238F** |
| **A181Q+H238F** | **A181Q+E185P** | **A181Q+R209I+H238F** |
| **A199E+H238F** | **E185P+A199E** | **A181Q+H238F+S239Q** |
| **H238F+A286V** | **E185P+A286V** | **N126Y+A181Q+E185P** |
| **H238F+L323F** | **E185P+L323F** | **T129I+A181Q+E185P** |
| **T116Q+N126Y+H238F** | **T116Q+N126Y+E185P** | **A181Q+E185P+N190F** |
| **N126Y+A181E+H238F** | **N126Y+A181E+E185P** | **A181Q+E185P+R209I** |
| **T116Q+T129I+H238F** | **T116Q+T129I+E185P** | |
| **T1291+A181E+H238F** | **T129I+A181E+E185P** | |
| **T116Q+N190F+H238F** | **T116Q+E185P+N190F** | |
| **A181E+N190F+H238F** | **A181E+E185P+N190F** | |
| **T116Q+R209I+H238F** | **T116Q+E185P+R209I** | |
| **A181E+R209I+H238F** | **A181E+E185P+R209I** | |
| **T116Q+H238F+S239Q** | **E185P+A275N+V277T** | |
| **A181E+H238F+S239Q** | **N126Y+G178H+E185P** | |
| **H238F+A275N+V277T** | **T129I+G178H+E185P** | |
| **N126Y+G178H+H238F** | **G178H+E185P+R209I** | |
| **T129I+G178H+H238F** | **G178H+E185P+S239Q** | |
| **G178H+N190F+H238F** | | |

Next, the following Table 2 shows more suitable mutation combinations which contribute to the enhancement of stability at a low pH and/or in the presence of a chelating agent. Therefore, variants having at least such mutation combinations are variants which particularly contribute to the enhancement of stability at a low pH and/or in the presence of a chelating agent.

**[Table 2]**

| |
|---|
| **N126Y+T129I+G178H+A181T+M197L+F203Y+R209I+H238F+S239Q+Y240F+E255T+Y358L+W406P+G474K** |
| **T116Q+N126Y+T129I+G178H+A181T+M197L+F203Y+R209I+H238F+S239Q+Y240F+E255T+Y358L+W406P+G474K** |
| **N126Y+T129I+G178H+A181T+M197L+A199E+F203Y+R209I+H238F+S239Q+Y240F+E255T+Y358L+W406P+G474K** |
| **T116Q+G178H+M197L+A199E+F203Y+R209I+H238F+S239Q+Y240F+E255T+Y358L+W406P+G474K** |
| **T116Q+N126Y+T1291+G178H+A181E+M197L+F203Y+R209I+H238F+S239Q+Y240F+E255T+Y358L+W406P+G474K** |
| **T116Q+N126Y+T1291+G178H+A181E+M197L+A199E+F203Y+R209I+H238F+S239Q+Y240F+E255T+Y358L+W406P+G474K** |
| **N126Y+T129I+G178H+A181E+M197L+A199E+F203Y+R209I+H238F+S239Q+Y240F+E255T+Y358L+W406P+G474K** |
| **T116Q+N126Y+T129I+G178H+A181E+M197L+F203Y+R209I+H238F+S239Q+Y240F+E255T+A275N+V277T+Y358L+W406P+G474K** |
| **T116Q+N126Y+T129I+G178H+A181E+M197L+A199E+F203Y+R209I+H238F+S239Q+Y240F+E255T+A286V+Y358L+W406P+G474K** |
| **T116Q+N126Y+T129I+G178H+A181E+M197L+F203Y+R209I+H238F+S239Q+Y240F+E255T+A275N+V277T+L323F+Y358L+W406P+G474K** |
| **T116Q+N126Y+T129I+G178H+A181E+M197L+F203Y+R209I+H238F+S239Q+Y240F+E255T+A286V+L323F+Y358L+W406P+G474K** |
| **T116Q+N126Y+T129I+G178H+A181E+M197L+A199E+F203Y+R209I+H238F+S239Q+Y240F+E255T+A275N+V277T+L323F+Y358L+W406P+G474K** |
| **T116Q+N126Y+T129I+G178H+A181E+M197L+A199E+F203Y+R209I+H238F+S239Q+Y240F+E255T+A286V+L323F+Y358L+W406P+G474K** |
| **N126Y+T129I+G178H+A181Q+M197L+F203Y+R209I+H238F+S2394+Y240F+E255T+Y358L+W406P+G474K** |
| **T116Q+N126Y+T129I+G178H+A181Q+M197L+F203Y+R209I+H238F+S239Q+Y240F+E255T+Y358L+W406P+G474K** |
| **N126Y+T129I+G178H+A181Q+M197L+A199E+F203Y+R209I+H238F+S239Q+Y240F+E255T+Y358L+W406P+G474K** |
| **T116Q+N126Y+T129I+G178H+A181Q+M197L+F203Y+R209I+H238F+S239Q+Y240F+E255T+A275N+V277T+Y358L+W406P+G474K** |
| **T116Q+N126Y+T129I+G178H+A181Q+M197L+F203Y+R209I+H238F+S239Q+Y240F+E255T+A275N+V277T+L323F+Y358L+W406P+G474K** |
| **T116Q+N126Y+T129I+G178H+A181Q+M197L+F203Y+R209I+H238F+S239Q+Y240F+E255T+A286V+L323F+Y358L+W406P+G474K** |
| **N126Y+T129I+G178H+A181Q+M197L+A199E+F203Y+R209I+H238F+S239Q+Y240F+E255T+A286V+Y358L+W406P+G474K** |
| **N126Y+T129I+G178H+A181Q+M197L+A199E+F203Y+R209I+H238F+S239Q+Y240F+E255T+A275N+V277T+L323F+Y358L+W406P+G474K** |
| **N126Y+T129I+G178H+A181Q+M197L+A199E+F203Y+R209I+H238F+S239Q+Y240F+E255T+A286V+L323F+Y358L+W406P+G474K** |

### <Polynucleotide encoding the variant of the present invention>

The variant of the present invention can be produced by using various mutagenesis techniques known in this technical field. For example, the variant of the present invention can be produced by mutating a polynucleotide encoding an amino acid residue to be modified in a parent α-amylase gene encoding its standard amino acid sequence (standard α-amylase gene) to a polynucleotide encoding the modified amino acid residue, and then allowing the mutated gene to express a variant.

The polynucleotide encoding the variant of the present invention can be in the form of single- or double-stranded DNA, RNA, or an artificial nucleic acid, or may be cDNA or chemically synthesized intron-free DNA.

In the present invention, as the means for mutating an amino acid residue of the parent α-amylase, various mutagenesis techniques known in this technical field can be used. For example, the polynucleotide encoding the variant of the present invention can be obtained by mutating, in a polynucleotide encoding the amino acid residue of the parent α-amylase (hereinafter also referred to as the "parent gene"), a nucleotide sequence encoding an amino acid residue to be mutated to a nucleotide sequence encoding the mutated amino acid residue.

Introduction of the target mutation into the parent gene can be basically performed by various site-specific mutagenesis methods well-known to a person skilled in the art. The site-specific mutagenesis method can be performed by any method such as an inverse PCR method or an annealing method. It is also possible to use commercially available site-specific mutagenesis kits (e.g., Stratagene's QuickChange II Site-Directed Mutagenesis Kit and QuickChange Multi Site-Directed Mutagenesis Kit, or the like).

Most commonly, site-specific mutagenesis of the parent gene can be carried out by using a mutation primer containing the nucleotide mutation to be introduced. The mutation primer may be designed to be annealed to a region containing a nucleotide sequence encoding an amino acid residue to be mutated in the parent gene, and to contain a nucleotide sequence having a nucleotide sequence (codon) encoding the mutated amino acid residue in place of the nucleotide sequence (codon) encoding the amino acid residue to be mutated. The nucleotide sequences (codons) encoding the amino acid residues before and after mutation can be appropriately recognized and selected by a person skilled in the art based on ordinary textbooks and the like. Alternatively, site-specific mutagenesis can also be performed by using a method in which DNA fragments, obtained by amplifying the upstream and downstream sides of the mutation site separately using two complementary primers containing the nucleotide mutation to be introduced, are linked into one by SOE (splicing by overlap extension)-PCR (Gene, 1989, 77 (1) : pp. 61-68) .

A template DNA containing the parent gene can be prepared by extracting a genome DNA from a microorganism producing the α-amylase described above by a standard method, or extracting an RNA and synthesizing a cDNA with reverse transcription. Alternatively, a corresponding nucleotide sequence may be chemically synthesized based on the amino acid sequence of the parent α-amylase, and used as the template DNA. A DNA sequence containing a base sequence encoding an α-amylase consisting of the amino acid sequence set forth in SEQ ID NO: 2 is shown in SEQ ID NO: 1, and a DNA sequence containing a base sequence encoding an α-amylase consisting of the amino acid sequence set forth in SEQ ID NO: 4 (YR288) is shown in SEQ ID NO: 3.

A mutation primer can be produced by well-known oligonucleotide synthesis methods such as the phosphoramidite method (Nucleic Acids R4esearch, 1989, 17: 7059-7071). Such primer synthesis can also be performed using, for example, a commercially available oligonucleotide synthesizer (e.g., ABI). Using a primer set containing the mutation primer, site-specific mutagenesis as described above can be carried out using the parent gene as the template DNA, thereby obtaining a polynucleotide encoding the variant of the present invention having the target mutation.

The polynucleotide encoding the variant of the present invention can contain single- or double-stranded DNA, cDNA, RNA, or other artificial nucleic acids. The DNA, cDNA, and RNA may be chemically synthesized. The polynucleotide may contain nucleotide sequences of untranslated regions (UTRs) in addition to open reading frames (ORFs). The codon of the polynucleotide may be optimized depending on the species of the transformant used for the production of the variant polynucleotide of the present invention. Information on codons used by various organisms is available from the Codon Usage Database ([www.kazusa.or.jp/codon/]).

### <Vector or DNA fragment>

The obtained polynucleotide encoding the variant of the present invention can be incorporated into a vector. The type of the vector to contain the polynucleotide is not particularly limited, and any vector such as a plasmid, a phage, a phagemid, a cosmid, a virus, a YAC vector or ashuttle vector, may be used. The vector is not limited, but is preferably a vector which can be amplified in bacteria, preferably Bacillus bacteria (e.g., *Bacillus subtilis* or variant strains thereof), and more preferably an expression vector which can induce the expression of transgenes in Bacillus bacteria. Among these, shuttle vectors, which are vectors replicable in Bacillus bacteria and any other organisms, can be preferably used in the recombinant production of the variant of the present invention. Examples of preferred vectors include, but are not limited to, pHA3040SP64, pHSP64R, or pASP64 (JP-B-3492935), pHY300PLK (an expression vector which can transform both *Escherichia coli* and *Bacillus subtilis;* Jpn J Genet, 1985, 60: 235-243), pAC3 (Nucleic Acids Res, 1988, 16: 8732), and other shuttle vectors; pUB110 (J Bacteriol, 1978, 134: 318-329), pTA10607 (Plasmid, 1987, 18: 8-15), and other plasmid vectors which can be used in the transformation of Bacillus bacteria; and the like. Other usable examples include *Escherichia* coli-derived plasmid vectors (e.g., pET22b(+), pBR322, pBR325, pUC57, pUC118, pUC119, pUC18, pUC19, and pBluescript, and the like).

The above vector may contain a DNA replication initiation region or a DNA region containing a replication origin. Alternatively, in the above vector, a regulatory sequence such as a promoter region for initiating the transcription of the gene, a terminator region or a secretion signal region for secreting the expressed protein outside the cell, may be operably linked to the upstream of the polynucleotide encoding the variant of the present invention (i.e., variant gene). The phrase that a gene and a regulatory sequence are "operably linked" means that the gene and the regulatory region are arranged so that the gene can be expressed under the control of the regulatory region.

The type of the regulatory sequence such as a promoter region, a terminator or a secretion signal region mentioned above is not particularly limited, and generally used promoters and secretion signal sequences can be appropriately selected depending on the host for introduction. Examples of preferred regulatory sequences that can be incorporated into the vector include the promoter, secretion signal sequence and the like of the cellulase gene of Bacllus sp. KSM-S237 strain.

Alternatively, a marker gene (e.g., a gene resistant to drugs such as ampicillin, neomycin, kanamycin and chloramphenicol) for selecting the host into which the vector of the present invention is appropriately introduced may be further incorporated into the vector. Alternatively, when an auxotroph is used as the host, a gene encoding the desired nutritional synthetic enzyme may be incorporated as a marker gene into the vector. Alternatively, when a selective culture medium in which a specific metabolism is required for growth, is used, a gene associated with the metabolism may be incorporated as a marker gene into the vector. Examples of such metabolism-related gene include acetamidase genes for utilizing acetamide as a nitrogen source.

The polynucleotide encoding the variant of the present invention, a regulatory sequence and a marker gene can be linked by a method known in the art, such as SOE (splicing by overlap extension)-PCR (Gene, 1989, 77: 61-68). Procedures for introducing the linked fragment into the vector are well known in the art.

### <Transformed cell>

The transformed cell of the present invention can be obtained by introducing a vector containing the polynucleotide encoding the variant of the present invention into a host or by introducing a DNA fragment containing the polynucleotide encoding the variant of the present invention into the genome of the host.

Examples of the host cells include microorganisms such as bacteria and filamentous fungi. Examples of bacteria include *Escherichia coli* and bacteria belonging to the genera Staphylococcus, Enterococcus, Listeria, and Bacillus. Preferred among these are *Escherichia coli* and Bacillus bacteria (e.g., *Bacillus subtilis* Marburg No. 168 (*Bacillus subtilis* 168 strain) or variant strains thereof). Examples of *Bacillus subtilis* variant strains include the nine-protease-deficient strain KA8AX described in J. Biosci. Bioeng., 2007, 104(2): 135-143, and the eight-protease-deficient strain with improved protein folding efficiency, D8PA strain, described in Biotechnol. Lett., 2011, 33(9): 1847-1852. Examples of filamentous fungi include Trichoderma, Aspergillus, Rizhopus, and the like.

Methods commonly used in the art, such as the protoplast method and the electroporation method, can be used to introduce the vector into the host. Strains into which the vector is appropriately introduced are selected by the marker gene expression, auxotrophyor the like as the index, whereby the desired transformant which the vector is introduced can be obtained.

Alternatively, a fragment obtained by linking the polynucleotide encoding the variant of the present invention, a regulatory sequence, and a marker gene can also be introduced directly into the genome of the host. For example, a DNA fragment in which sequences complementary to the genome of the host are added to both ends of the linked fragment is constructed by the SOE-PCR method or the like, and this DNA fragment is then introduced into the host to induce homologous recombination between the host genome and the DNA fragment, whereby the polynucleotide encoding the variant of the present invention is introduced into the genome of the host.

When the thus-obtained polynucleotide encoding the variant of the present invention, or a transformant into which a vector containing the polynucleotide is introduced, is cultured in a suitable culture medium, the gene encoding the protein on the vector is expressed to produce the variant of the present invention. The culture medium used for culturing the transformant can be appropriately selected by the person skilled in the art, depending on the type of microorganism of the transformant.

Alternatively, the variant of the present invention may be expressed from the polynucleotide encoding the variant of the present invention or a transcript thereof using a cell-free translation system. The "cell-free translation system" is such that reagents such as amino acids, necessary for the protein translation are added to a suspension obtained by mechanically destroying a cell, which serves as the host, to construct an in vitro transcription-translation system or an in vitro translation system.

The variant of the present invention produced in the above culture or cell-free translation system can be isolated or purified by using general methods used for protein purification, such as centrifugation, ammonium sulfate precipitation, gel chromatography, ion-exchange chromatography and affinity chromatography, singly or in a suitable combination. In this case, when the gene encoding the α-amylase variant of the present invention is operably linked to a secretion signal sequence on the vector in the transformant, the produced protein is secreted extracellularly, and can thereby be more easily collected from the culture. The protein collected from the culture may be further purified by known means.

The variant of the present invention thus obtained has enhanced stability at a low pH and/or in the presence of a chelating agent in comparison with the parent α-amylase.

The "enhanced stability at a low pH and/or in the presence of a chelating agent" means an enhanced ability to maintain α-amylase activity in a low-pH environment and/or in the presence of a chelating agent, in comparison with the parent α-amylase.

Here, the low pH refers to weak acidity, i.e., a pH of from 4 to 7, preferably a pH of from 4.5 to 6.5, and the stability in a low-pH environment means that the α-amylase activity is maintained in a cleansing composition having a pH of from about 4 to about 7, and that the α-amylase activity is maintained in cleaning water having a pH of from about 4 to about 7 prepared by dissolving or diluting the cleansing composition in water.

Examples of chelating agents include chelating agents which can be incorporated into cleansing compositions such as amino carboxylic acid-based chelating agents, phosphonic acid-based chelating agents, hydroxycarboxylic acid-based chelating agents and polyhydric carboxylic acid-based chelating agents, described later.

The stability at a low pH and/or in the presence of a chelating agent can be evaluated by using a method well known in the art. For example, an enzyme solution is added to a cleaning composition having a pH of from 4 to 7 containing a chelating agent, the α-amylase activity is measured before and after treatment for a predetermined time, the activity value of the sample before treatment is regarded as the initial activity, and the ratio of the activity value after treatment to the initial activity is regarded as the remaining activity (%).

The variant of the present invention is useful as an enzyme to be incorporated into various cleansing compositions, and particularly useful as an enzyme to be incorporated into acidic and chelating agent-incorporated cleansing compositions suitable for low-temperature cleaning at 40°C or lower.

The amount of the variant of the present invention incorporated into the cleansing composition is not particularly limited as long as the protein can exhibit activity. For example, the amount of the protein per kg of the cleansing composition is preferably 1 mg or more, more preferably 10 mg or more, and even more preferably 50 mg or more, as well as preferably 5 000 mg or less, more preferably 1 000 mg or less, and even more preferably 500 mg or less. The amount of the protein is also preferably from 1 to 5 000 mg, more preferably from 10 to 1 000 mg, and even more preferably from 50 to 500 mg.

In the cleansing composition, various enzymes can be used in combination in addition to the variant of the present invention. Examples include hydrolases, oxidases, reductases, transferases, lyases, isomerases, ligases, synthetases, and the like. Preferred among them are amylases different from the protein of the present invention, proteases, cellulases, keratinases, esterases, cutinases, lipases, pullulanases, pectinases, mannanases, glucosidases, glucanases, cholesterol oxidases, peroxidases, laccases, and the like; and particularly preferred are proteases, cellulases, amylases, and lipases.

Examples of proteases include commercially available Alcalase, Esperase, Everlase, Savinase, Kannase, and Progress Uno (registered trademarks; Novozymes A/S), PREFERENZ, EFFECTENZ, and EXCELLENZ (registered trademarks; DuPont), Lavergy (registered trademark; BASF), KAP (Kao Corporation) and the like.

Examples of cellulases include Celluclean and Carezyme (registered trademarks; Novozymes A/S); KAC, the alkaline cellulase produced by Bacillus sp. KSM-S237 strain described in JP-A-10-313859 and the variant alkaline cellulase described in JP-A-2003-313592 (Kao Corporation); and the like.

Examples of amylases include Termamyl, Duramyl, Stainzyme, Stainzyme Plus, and Amplify Prime (registered trademarks; Novozymes A/S), PREFERENZ and EFFECTENZ (registered trademarks; DuPont), KAM (Kao Corporation)and the like.

Examples of lipases include Lipolase and Lipex (registered trademarks; Novozymes A/S) and the like.

Known cleaning agent components can be incorporated into the cleansing composition, and examples of such known cleaning agent components include the following.

### (1) Surfactant

The surfactant is incorporated in an amount of from 0.5 to 60 mass% in the cleansing composition, and preferably from 10 to 45 mass% particularly in a powder cleansing composition, and from 20 to 90 mass% in a liquid cleansing composition. When the cleansing composition of the present invention is a clothing cleaning agent for laundry or a cleaning agent for an automatic dishwasher, the surfactant is generally incorporated in an amount of from 1 to 10 mass%, preferably from 1 to 5 mass%.

Examples of the surfactant used in the cleansing composition include one or a combination of anionic surfactants, non-ionic surfactants, amphoteric surfactants, and cationic surfactants; and anionic surfactants and non-ionic surfactants are preferred.

Examples of preferred anionic surfactants include sulfate ester salts of alcohols having from 10 to 18 carbon atoms, sulfate ester salts of alkoxylated alcohols having from 8 to 20 carbon atoms, alkylbenzene sulfonate, paraffin sulfonate, α-olefin sulfonate, internal olefin sulfonate, α-sulfo fatty acid salts, α-sulfo fatty acid alkyl ester salts, and fatty acid salts. In the present invention, particularly preferred is one or more anionic surfactants selected from the group consisting of linear alkylbenzene sulfonate with an alkyl chain having from 10 to 14 carbon atoms, more preferably from 12 to 14 carbon atoms, and internal olefin sulfone with an alkylene chain having from 12 to 20 carbon atoms, more preferably from 16 to 18 carbon atoms. Alkali metal salts and amines are preferable as counterions, and sodium and/or potassium, monoethanolamine, and diethanolamine are particularly preferred. For internal olefin sulfonic acid, reference can be made to, for example, WO 2017/098637.

Preferred non-ionic surfactants are polyoxyalkylene alkyl (from 8 to 20 carbon atoms) ether, alkyl polyglycoside, polyoxyalkylene alkyl (from 8 to 20 carbon atoms) phenyl ether, polyoxyalkylene sorbitan fatty acid (from 8 to 22 carbon atoms) ester, polyoxyalkylene glycol fatty acid (from 8 to 22 carbon atoms) ester and polyoxyethylene polyoxypropylene block polymers. In particular, preferred non-ionic surfactants are polyoxyalkylene alkyl ethers obtained by adding 4 to 20 moles of alkylene oxides such as ethylene oxide and propylene oxide to alcohols having from 10 to 18 carbon atoms [an HLB value (calculated by the Griffin method) of from 10.5 to 15.0, and preferably from 11.0 to 14.5].

### (2) Divalent metal ion scavenger

The divalent metal ion scavenger is incorporated in an amount of from 0.01 to 50 mass%, and preferably from 5 to 40 mass%. Examples of the divalent metal ion scavenger used in the cleansing composition of the present invention include condensed phosphates such as tripolyphosphates, pyrophosphates, and orthophosphates; aluminosilicates such as zeolites; synthetic layered crystalline silicates, nitrilotriacetates, ethylenediaminetetraacetates, citrates, isocitrates, polyacetal carboxylates and the like. Among these, crystalline aluminosilicates (synthetic zeolites) are particularly preferred. Among A-, X- and P-type zeolites, A-type zeolites are particularly preferred. As synthetic zeolites, those having an average primary particle size of from 0.1 to 10 µm, particularly from 0.1 to 5 µm, are preferably used.

### (3) Alkali agent

The alkali agent is incorporated in an amount of from 0.01 to 80 mass%, preferably from 1 to 40 mass%. In the case of powder cleaning agents, examples of alkali agents include alkali metal carbonates such as sodium carbonate, collectively called dense ash or light ash; and amorphous alkali metal silicates such as JIS Nos. 1, 2 and 3. These inorganic alkali agents are effective in the formation of the particle skeleton during cleaning agent drying, and relatively hard cleaning agents having excellent flowability can be obtained. Examples of other alkalis include sodium sesquicarbonate, sodium hydrogencarbonate, and the like. In addition, phosphates such as tripolyphosphates, also have the action as alkali agents. As alkali agents used in liquid cleaning agents, sodium hydroxide and mono-, di-, or triethanolamine can be used, in addition to the alkali agents mentioned above, and they can also be used as counterions of activators.

### (4) Anti-recontamination agent

The anti-recontamination agent is incorporated in an amount of from 0.001 to 10 mass%, preferably from 1 to 5 mass%. Examples of the anti-recontamination agent used in the cleansing composition of the present invention include polyethylene glycol, carboxylic acid-based polymers, polyvinyl alcohol, polyvinylpyrrolidone and the like. Among these, carboxylic acid-based polymers have the function of scavenging metal ions and the ability to disperse solid particle stains from the clothing into the laundry bath, as well as the anti-recontamination ability. Carboxylic acid-based polymers are homopolymers or copolymers of acrylic acid, methacrylic acid, itaconic acid, or the like. Preferred copolymers are copolymers of the above monomers and maleic acid, and those with a molecular weight of from several thousands to a hundred thousand are preferred. In addition to the carboxylic acid-based polymers mentioned above, polymers such as polyglycidylates, cellulose derivatives such as carboxymethyl cellulose, and amino carboxylic acid-based polymers such as polyaspartic acid are also preferred because they have metal ion scavengers, dispersants, and anti-recontamination ability.

### (5) Bleaching agent

The bleaching agent such as hydrogen peroxide or percarbonate, is preferably incorporated in an amount of from 1 to 10 mass%. When the bleaching agent is used, tetraacetylethylenediamine (TAED) or the bleach activator described in JP-A-6-316700 can be incorporated in an amount of from 0.01 to 10 mass%.

### (6) Fluorescent agent

Examples of the fluorescent agent used in the cleansing composition include biphenyl-type fluorescent agents (e.g., Tinopal CBS-X and the like) and stilbene-type fluorescent agents (e.g., a DM-type fluorescent dye and the like). The fluorescent agent is preferably incorporated in an amount of from 0.001 to 2 mass%.

### (7) Chelating agent

A chelating agent is incorporated in order to, for example, facilitate the cleaning of stains and reduce the water hardness during cleaning.

Examples of such chelating agents include amino carboxylic acid-based chelating agents, phosphonic acid-based chelating agents, hydroxycarboxylic acid-based chelating agents, polyhydric carboxylic acid-based chelating agents, and the like.

Examples of amino carboxylic acid-based chelating agents include ethylenediaminetetraacetic acid (EDTA), nitrilotriacetic acid (NTA), hydroxyethylethylenediaminetriacetic acid (HEDTA), diethylenetriaminepentaacetic acid (DTPA), methylglycinediacetic acid (MGDA), triethylenetetraminehexaacetic acid (TTHA), glutamic acid diacetic acid (GLDA), hydroxyethyliminodiacetic acid (HIDA), dihydroxyethylglycine (DHEG), aspartic acid diacetic acid (ASDA), ethylenediaminesuccinic acid (EDDS) and salts thereof.

Examples of phosphonic acid-based chelating agents include hydroxyethylidene diphosphonic acid (HEDP), nitrilotrismethylenephosphonic acid (NTMP), phosphonobutanetricarboxylic acid (PBTC), ethylenediaminetetramethylenephosphonic acid (EDTMP) and salts thereof.

Examples of hydroxycarboxylic acid-based chelating agents include citric acid, malic acid, tartaric acid, gluconic acid, lactic acid, and salts thereof.

Examples of polyhydric carboxylic acid-based chelating agents include succinic acid, oxalic acid, glutaric acid, adipic acid, fumaric acid, malonic acid, and salts thereof.

### (8) Buffer

A buffer is incorporated in order to form a buffer system capable of maintaining the low pH of the cleaning liquid. Examples of such buffers include mixtures of organic acids and salts thereof, such as mixtures of polycarboxylic acids and salts thereof, preferably a mixture of citric acid and citrate.

### (9) Other components

The cleansing composition may contain builders, softeners, reducing agents (e.g., sulfite), foam inhibitors (e.g., silicone), fragrances, antibacterial and antifungal agents (e.g., Proxel [trade name] and benzoic acid), and other additives known in the field of clothing cleaning agents.

The cleansing composition can be produced in accordance with a standard method by combining the protein of the present invention obtained by the above method and the known cleaning agent components mentioned above. The form of the cleaning agent can be selected depending on the application. For example, the cleaning agent can be in the form of liquid, powder, granules, paste, solids or the like.

The thus-obtained cleansing composition can be used as a clothing cleaning agent, a dishwashing cleaning agent, a bleaching agent, a cleaning agent for hard surface cleaning, a drain cleaning agent, a denture cleaning agent, a disinfecting cleaning agent for medical instruments, or the like; preferably a clothing cleaning agent and a dishwashing cleaning agent; and more preferably a clothing cleaning agent for laundry (laundry cleaning agent), a dishwashing cleaning agent for hand washing, and a cleaning agent for an automatic dishwasher.

The cleansing composition is also suitable for use at a low temperature (e.g., 40°C or lower, 35°C or lower, 30°C or lower, or 25°C or lower and at 5°C or higher, 10°C or higher, or 15°C or higher).

Regarding the embodiments described above, the present invention further discloses the following aspects.
<1> A variant of a parent α-amylase, comprising an amino acid residue substitution at a position corresponding to position H238 or E185 of an amino acid sequence set forth in SEQ ID NO: 2, and an amino acid residue substitution shown in the following (A) or (B),
   the parent α-amylase or α-amylase variant having at least 80% sequence identity to the amino acid sequence set forth in SEQ ID NO: 2 (excluding α-amylase variants having amino acid residue substitutions at three positions corresponding to positions H238, S239 and G178, three positions corresponding to positions H238, R209 and G178, and three positions corresponding to positions E185, N190 and G178 of the same amino acid sequence): (A) an amino acid residue substitution at one or more positions selected from the group consisting of positions corresponding to positions T116, A181, A199, A275, V277, A286 and L323 of the amino acid sequence set forth in SEQ ID NO: 2; or
   (B) an amino acid residue substitution at a position corresponding to position G178 of the amino acid sequence set forth in SEQ ID NO: 2, and an amino acid residue substitution at one or more positions selected from the group consisting of positions corresponding to positions N126, T129, N190, R209 and S239 of the same amino acid sequence.
<2> The variant according to <1>, wherein the amino acid residue substitution shown in (A) further comprise an amino acid residue substitution at one or more positions selected from the group consisting of positions corresponding to positions N126, T129, N190, R209, S239, G178, M197, F203, Y240, E255, Y358, W406 and G474 of the amino acid sequence set forth in SEQ ID NO: 2.
<3> The variant according to <1> or <2>, comprising two or more amino acid residue substitutions at two or more positions selected from the group consisting of positions corresponding to positions T116, A181, A199, A275, V277, A286 and L323 of the amino acid sequence set forth in SEQ ID NO: 2 shown in (A).
<4> The variant according to any one of <1> to <3>, wherein the amino acid residue substitutions at positions corresponding to positions H238, E185, T116, A181, A199, A275, V277, A286, L323, G178, N126, T129, N190, R209 and S239 are H238F, E185P, T116Q, A181T/E/Q, A199E, A275N, V277T, A286V, L323F, G178H, N126Y, T129I, N190F, R209V/L/I and S239A/Q/D/L/Y/P/H, respectively.
<5> The variant according to <1>, wherein the variant comprises at least mutations selected from the group consisting of mutation combinations shown in the above Table 1-1, Table 1-2, Table 1-3 and Table 2:
   <6> A polynucleotide encoding the variant according to any one of <1> to <5>.
   <7> A vector or DNA fragment comprising the polynucleotide according to <6>.
   <8> A transformed cell comprising the vector or DNA fragment according to <7>.
   <9> The transformed cell according to <8>, which is a microorganism.
   <10> A cleansing composition comprising the variant according to any one of <1> to <5>.
   <11> The cleansing composition according to <10>, which is a clothing cleaning agent or a dishwashing cleaning agent.
   <12> The cleansing composition according to <11>, which is a powder or a liquid.
   <13> The cleansing composition according to <11> or <12>, which is weakly acidic, preferably has a pH of from 4 to 7.
   <14> The cleansing composition according to any one of <11> to <13>, which comprises a chelating agent.
   <15> The cleansing composition according to <14>, wherein the chelating agent is an amino carboxylic acid-based chelating agent, a phosphonic acid-based chelating agent, a hydroxycarboxylic acid-based chelating agent, or a polyhydric carboxylic acid-based chelating agent.

### Examples

### (1) Construction of YR288 variant expression plasmid

The method for constructing YR288 variant described in the following examples is shown below. A forward primer having 15 bases of a sequence complementary to a reverse primer at the 5'-terminal and containing a variant sequence, and the reverse primer having a base just before the variant sequence at the 5'-terminal were used as a mutagenesis primer pair. The YR288 R178Δ T180Δ expression plasmid described in the examples of JP-A-2022-019601 or the YR288 variant expression plasmid produced in this example was used as a template, and PCR was performed by using the mutagenesis primer pair. When multiple fragments were linked, using each of the PCR products, the In-Fusion reaction was performed in accordance with the protocol of the In-Fusion, HD Cloning kit (Clontech). With the PCR product or In-Fusion reaction solution, *Bacillus subtilis* was transformed by the protoplast method to obtain a transformant retaining the target YR288 variant expression plasmid.

### (2) Enzyme production culture

The recombinant *Bacillus subtilis* colonies obtained in (1) were inoculated in a 96-deep-well plate into which 500 µL of LB culture medium supplemented with 10 ppm tetracycline was dispensed, and then cultured at 32°C at 1 500 rpm overnight. Next day, 20 µL of the culture was inoculated in a 96-deep-well plate into which 500 µL of 2xL-maltose culture medium (2% tryptone, 1% yeast extract, 1% NaCl, 7.5% maltose, 7.5 ppm manganese sulfate pentahydrate, 0.04% calcium chloride dihydrate, and 10 ppm tetracycline; % denotes (w/v)%) was dispensed, and cultured at 32°C at 1 500 rpm for 2 days. Then, the culture supernatant containing the enzyme produced from the bacterial body was collected by centrifugation and used as an enzyme solution.

### (3) Measurement of protein concentration of culture supernatant

For the measurement of the protein concentration of the culture supernatant, Protein Assay Rapid Kit Wako II (FUJIFILM Wako Pure Chemical Corporation) was used. The protein concentration of a culture supernatant of pHY300PLK (Takara Bio Inc.)-introduced strain without an amylase expression cassette was used as a blank to calculate the amylase concentration of the culture supernatant.

### (4) Evaluation of the amylase variant stability (1)

Various variants were constructed by the method described in Example (1) using YR288 R178Δ+T180Δ (SEQ ID NO: 2) as a parent polypeptide, and the storage stability of these variants under acidic conditions and in the presence of a high chelating agent was evaluated.

An amylase solution was added to a solution prepared by mixing 200 mM EDTA-2NA and 50 mM Britton-Robinson buffer (pH: 5.0) in equal amounts, followed by incubation at 40°C for 30 minutes, and the resultant was then 10-fold diluted with 50 mM Britton-Robinson buffer (pH: 8.0) and used to measure the activity. The activity value of the sample before the treatment at 40°C was used as the initial activity, and the ratio of the activity value after treatment to the initial activity was regarded as the remaining activity (%). Table 3 shows the results. All of the variants showed high stability under acidic conditions and in the presence of a chelating agent.

**[Table 3]**

| **Variant** | **Remaining activity (%)** |
|---|---|
| **SEQ ID NO: 1 (template)** | **0.3** |
| **T116Q+H238F** | **15.1** |
| **A181T+H238F** | **17.3** |
| **A181E+H238F** | **18** |
| **A181Q+H238F** | **16.3** |
| **A199E+H238F** | **17.1** |
| **H238F+A286V** | **20.1** |
| **H238F+L323F** | **22.1** |
| **T116Q+N126Y+H238F** | **22.9** |
| **N126Y+A181E+H238F** | **24.9** |
| **T116Q+T129I+H238F** | **20** |
| **T1291+A181E+H238F** | **21.5** |
| **T116Q+N190F+H238F** | **29.7** |
| **A181E+N190F+H238F** | **34.3** |
| **T116Q+R209I+H238F** | **19.8** |
| **A181E+R209I+H238F** | **23.5** |
| **T116Q+H238F+S239Q** | **44.1** |
| **A181E+H238F+S239Q** | **44.2** |
| **H238F+A275N+V277T** | **20.6** |
| **N126Y+G178H+H238F** | **30.6** |
| **T129I+G178H+H238F** | **22.3** |
| **G178H+N190F+H238F** | **40.5** |
| **N126Y+A181Q+H238F** | **22.8** |
| **T129I+A181Q+H238F** | **23** |
| **A181Q+R209I+H238F** | **22.5** |
| **A181Q+H238F+S239Q** | **7.9** |

### (5) Evaluation of the amylase variant stability (2)

An amylase solution was added to AATCC High Efficiency Standard Reference Detergent WITHOUT Brightener 10-fold diluted with 100 mM citrate buffer (pH: 5.0), followed by incubation at 50°C for 1 hour, and the resultant was then 10-fold diluted with 50 mM Britton-Robinson buffer (pH: 8.0) and used to measure the activity. The activity value of the sample before the treatment at 50°C was used as the initial activity, and the ratio of the activity value after treatment to the initial activity was regarded as the remaining activity (%). Table 4 (Table 4-1, Table 4-2, and Table 4-3) shows the results. All of the variants showed high stability in the diluted cleaning agents.

**[Table 4]**

| **Table 4-1** | |
|---|---|
| **Variant** | **Remaining activity (%)** |
| **SEQ ID NO: 1 (template)** | **0.1** |
| **T116Q+H238F** | **5.4** |
| **A181T+H238F** | **9.1** |
| **A181E+H238F** | **16.8** |
| **A181Q+H238F** | **9.4** |
| **A199E+H238F** | **16.4** |
| **H238F+A286V** | **16.4** |
| **H238F+L323F** | **21.3** |
| **T116Q+N126Y+H238F** | **13.1** |
| **N126Y+A181E+H238F** | **21** |
| **T116Q+T129I+H238F** | **16** |
| **T1291+A181E+H238F** | **21.3** |
| **T116Q+N190F+H238F** | **28.9** |
| **A181E+N190F+H238F** | **42.2** |
| **T116Q+R209I+H238F** | **13.3** |
| **A181E+R209I+H238F** | **24.8** |
| **T116Q+H238F+S239Q** | **48.5** |
| **A181E+H238F+S239Q** | **54.7** |
| **H238F+A275N+V277T** | **14.5** |
| **N126Y+G178H+H238F** | **29.7** |
| **T129I+G178H+H238F** | **23.6** |
| **G178H+N190F+H238F** | **48.6** |

| **Table 4-2** | |
|---|---|
| **Variant** | **Remaining activity (%)** |
| **SEQ ID NO:1 (template)** | **0.1** |
| **T116Q+E185P** | **22.8** |
| **A181T+E185P** | **22.1** |
| **A181E+E185P** | **37.3** |
| **A181Q+E185P** | **25.7** |
| **E185P+A199E** | **29.7** |
| **E185P+A286V** | **43.9** |
| **E185P+L323F** | **28.1** |
| **T116Q+N126Y+E185P** | **38.6** |
| **N126Y+A181E+E185P** | **58.1** |
| **T116Q+T129I+E185P** | **34.3** |
| **T129I+A181E+E185P** | **54.8** |
| **T116Q+E185P+N190F** | **62.1** |
| **A181E+E185P+N190F** | **70.1** |
| **T116Q+E185P+R209I** | **35** |
| **A181E+E185P+R209I** | **54.4** |
| **E185P+A275N+V277T** | **29.8** |
| **N126Y+G178H+E185P** | **62.7** |
| **T129I+G178H+E185P** | **57** |
| **G178H+E185P+R209I** | **57.2** |
| **G178H+E185P+S239Q** | **55.6** |

| **Table 4-3** | |
|---|---|
| **Variant** | **Remaining activity (%)** |
| **SEQ ID NO: 1 (template)** | **0** |
| **N126Y+A181Q+H238F** | **14.5** |
| **T1291+A181Q+H238F** | **24.3** |
| **A181Q+N190F+H238F** | **42.2** |
| **A181Q+R209I+H238F** | **19.5** |
| **A181Q+H238F+S239Q** | **10.3** |
| **N126Y+A181Q+E185P** | **40.1** |
| **T1291+A181Q+E185P** | **39.9** |
| **A181Q+E185P+N190F** | **61.9** |
| **A181Q+E185P+R209I** | **42.3** |

### (6) Evaluation of the amylase variant stability (3)

An amylase solution was added to AATCC High Efficiency Standard Reference Detergent WITHOUT Brightener 10-fold diluted with 100 mM citrate buffer (pH: 5.0), followed by incubation at 50°C for 18 hours, and the resultant was then 10-fold diluted with 50 mM Britton-Robinson buffer (pH: 8.0) and used to measure the activity. The activity value of the sample before the treatment at 50°C was used as the initial activity, and the ratio of the activity value after treatment to the initial activity was regarded as the remaining activity (%). Table 5 (Table 5-1 and Table 5-2) shows the results. All of the variants showed high stability in the diluted cleaning agents.

### (7) Evaluation of the amylase variant stability (4)

A 50% w/w citric acid solution was added to AATCC High Efficiency Standard Reference Detergent WITHOUT Brightener so that the final concentration would be 100 mM, and the pH was adjusted to 5.2 with 1M NaOH. An amylase solution was added to this cleaning agent, followed by incubation at 40°C for 1 week, and the resultant was then 10-fold diluted with 50 mM Britton-Robinson buffer (pH: 8.0) and used to measure the activity. The activity value of the sample before the treatment at 40°C was used as the initial activity, and the ratio of the activity value after treatment to the initial activity was regarded as the remaining activity (%). Table 6 shows the results. All of the variants showed high stability in the cleaning agents.

**[Table 5]**

| **Table 5-1** | |
|---|---|
| **Variant** | **Remaining activity (%)** |
| **SEQ ID NO: 1 (template)** | **0** |
| **T116Q+N126Y+T129I+G178H+A181T+M197L+F203Y+R209I+H238F+S239Q+Y240F+E255T+Y358L+W406P+G474K** | **33.1** |
| **N126Y+T129I+G178H+A181T+M197L+A199E+F203Y+R209I+H238F+S239Q+Y240F+E255T+Y358L+W406P+G474K** | **41.4** |
| **T116Q+G178H+M197L+A199E+F203Y+R209I+H238F+S239Q+Y240F+E255T+Y358L+W406P+G474K** | **51.3** |
| **T116Q+N126Y+T129I+G178H+A181E+M197L+F203Y+R209I+H238F+S239Q+Y240F+E255T+Y358L+W406P+G474K** | **58.1** |
| **T116Q+N126Y+T129I+G178H+A181E+M197L+F203Y+R209I+H238F+S239Q+Y240F+E255T+A275N+V277T+Y358L+W406P+G474K** | **60.7** |
| **N126Y+T129I+G178H+A181E+M197L+A199E+F203Y+R209I+H238F+S239Q+Y240F+E255T+Y358L+W406P+G474K** | **61** |
| **T116Q+N126Y+T129I+G178H+A181E+M197L+A199E+F203Y+R209I+H238F+S239Q+Y240F+E255T+Y358L+W406P+G474K** | **62.5** |
| **T116Q+N126Y+T129I+G178H+A181E+M197L+F203Y+R209I+H238F+S239Q+Y240F+E255T+A286V+L323F+Y358L+W406P+G474K** | **62.3** |
| **T116Q+N126Y+T129I+G178H+A181E+M197L+A199E+F203Y+R209I+H238F+S239Q+Y240F+E255T+A275N+V277T+L323F+Y358L+W406P+G474K** | **62.4** |
| **T116Q+N126Y+T129I+G178H+A181E+M197L+A199E+F203Y+R209I+H238F+S239Q+Y240F+E255T+A286V+L323F+Y358L+W406P+G474K** | **64.8** |
| **T116Q+N12SY+T129I+G178H+A181E+M197L+F203Y+R209I+H238F+S239Q+Y240F+E255T+A275N+V277T+L323F+Y358L+W406P+G474K** | **67.6** |

**Table 5-2**

| **Variant** | **Remaining activity (%)** |
|---|---|
| **SEQ ID NO: 1 (template)** | **0.1** |
| **N126Y+T129I+G178H+A181Q+M197L+F203Y+R209I+H238F+S239Q+Y240F+E255T+Y358L+W406P+G474K** | **49.7** |
| **T116Q+N126Y+T129I+G178H+A181Q+M197L+F203Y+R209I+H238F+S239Q+Y240F+E255T+Y358L+W406P+G474K** | **51.2** |
| **N126Y+T129I+G178H+A181Q+M197L+A199E+F203Y+R209I+H238F+S239Q+Y240F+E255T+Y358L+W406P+G474K** | **56.3** |
| **T116Q+N126Y+T129I+G178H+A181Q+M197L+F203Y+R209I+H238F+S239Q+Y240F+E255T+A275N+V277T+Y358L+W406P+G474K** | **53.6** |
| **T116Q+N126Y+T129I+G178H+A181Q+M197L+F203Y+R209I+H238F+S239Q+Y240F+E255T+A275N+V277T+L323F+Y358L+W406P+G474K** | **55.4** |
| **T116Q+N126Y+T1291+G178H+A181Q+M197L+F203Y+R2091+H238F+S239Q+Y240F+E255T+A286V+L323F+Y358L+W406P+G474K** | **62.7** |
| **N126Y+T129I+G178H+A181Q+M197L+A199E+F203Y+R209I+H238F+S239Q+Y240F+E255T+A286V+Y358L+W406P+G474K** | **62.1** |
| **N126Y+T129I+G178H+A181Q+M197L+A199E+F203Y+R209I+H238F+S239Q+Y240F+E255T+A275N+V277T+L323F+Y358L+W406P+G474K** | **67.4** |
| **N126Y+T129I+G178H+A181Q+M197L+A199E+F203Y+R209I+H238F+S239Q+Y240F+E255T+A286V+L323F+Y358L+W406P+G474K** | **61.5** |

**[Table 6]**

| **Variant** | **Remaining activity (%)** |
|---|---|
| **SEQ ID NO: 1 (template)** | **0.2** |
| **N126Y+T129I+G178H+A181T+M197L+F203Y+R209I+H238F+S239Q+Y240F+E255T+Y358L+W406P+G474K** | **21.8** |
| **T116Q+G178H+M197L+A199E+F203Y+R209I+H238F+S239Q+Y240F+E255T+Y358L+W406P+G474K** | **31.1** |
| **T116Q+N126Y+T129I+G178H+A181E+M197L+F203Y+R209I+H238F+S239Q+Y240F+E255T+Y358L+W406P+G474K** | **33.5** |
| **T116Q+N126Y+T129I+G178H+A181E+M197L+F203Y+R209I+H238F+S239Q+Y240F+E255T+A275N+V277T+Y358L+W406P+G474K** | **39.4** |
| **T116Q+N126Y+T129I+G178H+A181E+M197L+A199E+F203Y+R209I+H238F+S239Q+Y240F+E255T+A286V+L323F+Y358L+W406P+G474K** | **45.5** |
| **T116Q+N126Y+T129I+G178H+A181E+M197L+F203Y+R209I+H238F+S239Q+Y240F+E255T+A275N+V277T+L323F+Y358L+W406P+G474K** | **48** |
| **T116Q+N126Y+T129I+G178H+A181E+M197L+A199E+F203Y+R209I+H238F+S239Q+Y240F+E255T+A286V+Y358L+W406P+G474K** | **53** |

## Claims

1. A variant of a parent α-amylase, comprising an amino acid residue substitution at a position corresponding to position H238 or E185 of an amino acid sequence set forth in SEQ ID NO: 2, and an amino acid residue substitution shown in the following (A) or (B),
the parent α-amylase or α-amylase variant having at least 80% sequence identity to the amino acid sequence set forth in SEQ ID NO: 2 (excluding α-amylase variants having amino acid residue substitutions at three positions corresponding to positions H238, S239 and G178, three positions corresponding to positions H238, R209 and G178, and three positions corresponding to positions E185, N190 and G178 of the same amino acid sequence):
(A) an amino acid residue substitution at one or more positions selected from the group consisting of positions corresponding to positions T116, A181, A199, A275, V277, A286 and L323 of the amino acid sequence set forth in SEQ ID NO: 2; or
(B) an amino acid residue substitution at a position corresponding to position G178 of the amino acid sequence set forth in SEQ ID NO: 2, and an amino acid residue substitution at one or more positions selected from the group consisting of positions corresponding to positions N126, T129, N190, R209 and S239 of the same amino acid sequence.

2. The variant according to Claim 1, wherein the acid residue substitutions shown in (A) further comprise an amino acid residue substitution at one or more positions selected from the group consisting of positions corresponding to positions N126, T129, N190, R209, S239, G178, M197, F203, Y240, E255, Y358, W406 and G474 of the amino acid sequence set forth in SEQ ID NO: 2.

3. The variant according to Claim 1 or 2, comprising two or more amino acid residue substitutions at two or more positions selected from the group consisting of positions corresponding to positions T116, A181, A199, A275, V277, A286 and L323 of the amino acid sequence set forth in SEQ ID NO: 2 shown in (A).

4. The variant according to any one of Claims 1 to 3, wherein the amino acid residue substitutions at positions corresponding to positions H238, E185, T116, A181, A199, A275, V277, A286, L323, G178, N126, T129, N190, R209 and S239 are H238F, E185P, T116Q, A181T/E/Q, A199E, A275N, V277T, A286V, L323F, G178H, N126Y, T129I, N190F, R209V/L/I and S239A/Q/D/L/Y/P/H, respectively.

5. The variant according to Claim 1, wherein the variant comprises at least mutations selected from the group consisting of mutation combinations shown in the following Table 1-1, Table 1-2, Table 1-3 and Table 2:
| **Table 1-1** | **Table 1-2** | **Table 1-3** |
|---|---|---|
| **T116Q+H238F** | **T116Q+E185P** | **N126Y+A181Q+H238F** |
| | | |
|---|---|---|
| **A181T+H238F** | **A181T+E185P** | **T129I+A181Q+H238F** |
| **A181E+H238F** | **A181E+E185P** | **A181Q+N190F+H238F** |
| **A181Q+H238F** | **A181Q+E185P** | **A181Q+R209I+H238F** |
| **A199E+H238F** | **E185P+A199E** | **A181Q+H238F+S239Q** |
| **H238F+A286V** | **E185P+A286V** | **N126Y+A181Q+E185P** |
| **H238F+L323F** | **E185P+L323F** | **T129I+A181Q+E185P** |
| **T116Q+N126Y+H238F** | **T116Q+N126Y+E185P** | **A181Q+E185P+N190F** |
| **N126Y+A181E+H238F** | **N126Y+A181E+E185P** | **A181Q+E185P+R209I** |
| **T116Q+T129I+H238F** | **T116Q+T129I+E185P** | |
| **T129I+A181E+H238F** | **T129I+A181E+E185P** | |
| **T116Q+N190F+H238F** | **T116Q+E185P+N190F** | |
| **A181E+N190F+H238F** | **A181E+E185P+N190F** | |
| **T116Q+R209I+H238F** | **T116Q+E185P+R209I** | |
| **A181E+R209I+H238F** | **A181E+E185P+R209I** | |
| **T116Q+H238F+S239Q** | **E185P+A275N+V277T** | |
| **A181E+H238F+S239Q** | **N126Y+G178H+E185P** | |
| **H238F+A275N+V277T** | **T129I+G178H+E185P** | |
| **N126Y+G178H+H238F** | **G178H+E185P+R209I** | |
| **T129I+G178H+H238F** | **G178H+E185P+S239Q** | |
| **G178H+N190F+H238F** | | |
**[Table 2]**
| |
|---|
| N126Y+T129I+G178H+A181T+M197L+F203Y+R209I+H238F+S239Q+Y240F+E255T+Y358L+W406P+G474K |
| T116Q+N126Y+T129I+G178H+A181T+M197L+F203Y+R209I+H238F+S239Q+Y240F+E255T+Y358L+W406P+G474K |
| N126Y+T129I+G178H+A181T+M197L+A199E+F203Y+R209I+H238F+S239Q+Y240F+E255T+Y358L+W406P+G474K |
| T116Q+G178H+M197L+A199E+F203Y+R209I+H238F+S239Q+Y240F+E255T+Y358L+W406P+G474K |
| T116Q+N126Y+T129I+G178H+A181E+M197L+F203Y+R209I+H238F+S239Q+Y240F+E255T+Y358L+W406P+G474K |
| T116Q+N126Y+T129I+G178H+A181E+M197L+A199E+F203Y+R209I+H238F+S239Q+Y240F+E255T+Y358L+W406P+G474K |
| N116Y+T129I+G178H+A181E+M197L+A199E+F203Y+R209I+H238F+S239Q+Y240F+E255T+Y358L+W406P+G474K |
| T116Q+N126Y+T129I+G178H+A181E+M197L+F203Y+R209I+H238F+S239Q+Y240F+E255T+A275N+V277T+Y358L+W406P+G474K |
| T116Q+N126Y+T129I+G178H+A181E+M197L+A199E+F203Y+R209I+H238F+S239Q+Y240F+E255T+A286V+Y358L+W406P+G474K |
| T116Q+N126Y+T129I+G178H+A181E+M197L+F203Y+R209I+H238F+S239Q+Y240F+E255T+A275N+V277T+L323F+Y358L+W406P+G474K |
| T116Q+N126Y+T129I+G178H+A181E+M197L+F203Y+R209I+H238F+S239Q+Y240F+E255T+A286V+L323F+Y358L+W406P+G474K |
| T116Q+N126Y+T129I+G178H+A181E+M197L+A199E+F203Y+R209I+H238F+S239Q+Y240F+E255T+A275N+V277T+L323F+Y358L+W406P+G474K |
| T116Q+N126Y+T129I+G178H+A181E+M197L+A199E+F203Y+R209I+H238F+S239Q+Y240F+E255T+A286V+L323F+Y358L+W406P+G474K |
| N126Y+T129I+G178H+A181Q+M197L+F203Y+R209I+H238F+S239Q+Y240F+E255T+Y358L+W406P+G474K |
| T116Q+N126Y+T129I+G178H+A181Q+M197L+F203Y+R209I+H238F+S239Q+Y240F+E255T+Y358L+W406P+G474K |
| N126Y+T129I+G178H+A181Q+M197L+A199E+F203Y+R209I+H238F+S239Q+Y240F+E255T+Y358L+W406P+G474K |
| T116Q+N126Y+T129I+G178H+A181Q+M197L+F203Y+R209I+H238F+S239Q+Y240F+E255T+A275N+V277T+Y358L+W406P+G474K |
| T116Q+N126Y+T129I+G178H+A181Q+M197L+F203Y+R209I+H238F+S239Q+Y240F+E255T+A275N+V277T+L323F+Y358L+W406P+G474K |
| T116Q+N126Y+T129I+G178H+A181Q+M197L+F203Y+R209I+H238F+S239Q+Y240F+E255T+A286V+L323F+Y358L+W406P+G474K |
| N126Y+T129I+G178H+A181Q+M197L+A199E+F203Y+R209I+H238F+S239Q+Y240F+E255T+A286V+Y358L+W406P+G474K |
| N126Y+T129I+G178H+A181Q+M197L+A199E+F203Y+R209I+H238F+S239Q+Y240F+E255T+A275N+V277T+L323F+Y358L+W406P+G474K |
| N126Y+T129I+G178H+A181Q+M197L+A199E+F203Y+R209I+H238F+S239Q+Y240F+E255T+A286V+L323F+Y358L+W406P+G474K |

6. A polynucleotide encoding the variant according to any one of Claims 1 to 5.

7. A vector or DNA fragment comprising the polynucleotide according to Claim 6.

8. A transformed cell comprising the vector or DNA fragment according to Claim 7.

9. The transformed cell according to Claim 8, which is a microorganism.

10. A cleansing composition comprising the variant according to any one of Claims 1 to 5.

11. The cleansing composition according to Claim 10, which is a clothing cleaning agent or a dishwashing cleaning agent.

12. The cleansing composition according to Claim 11, which is a powder or a liquid.

13. The cleansing composition according to Claim 11 or 12, which is weakly acidic.

14. The cleansing composition according to any one of Claims 11 to 13, which comprises a chelating agent.
